# EUROPEAN PATENT SPECIFICATION

(11) **EP 3 888 721 B1**
(45) Date of publication and mention of the grant of the patent: **12.02.2025**
(21) Application number: 21166014.7
(22) Date of filing: 30.03.2021
(51) Int. Cl.: A61M 1/02, A61H 9/00, A61H 15/00, A61B 17/12, A61H 15/02, A61B 5/15, A61N 1/18, A61N 1/04, A61N 1/36

(54) **BLOOD FLOW ASSIST DEVICE**
VORRICHTUNG ZUR UNTERSTÜTZUNG DES BLUTFLUSSES
DISPOSITIF D'ASSISTANCE AU FLUX SANGUIN

(30) Priority: 01.04.2020 US 202063003470 P
(43) Date of publication of application: 06.10.2021
(73) Proprietor: Fenwal, Inc., Lake Zurich, IL 60047 (US)
(72) Inventor: CASE, Brian C., Lake Zurich, Illinois 60047 (US); MIN, Kyungyoon, Lake Zurich, Illinois 60047 (US); DUNCAN, Tammy, Lake Zurich, Illinois 60047 (US)
(74) Representative: Maikowski & Ninnemann Patentanwälte Partnerschaft mbB

(56) References cited:
- WO-A1-2018/052676
- WO-A1-2019/006349
- US-A- 4 883 462
- US-A1- 2017 181 921

## Description

### Field of the Disclosure

The present disclosure generally relates to systems and devices for drawing blood from a patient or donor. More particularly, the present disclosure relates to a blood flow assist device and system for ensuring proper blood flow from a patient or donor.

### Description of Related Art

Blood may be drawn from the arm or hand of a patient or donor for any of a number of reasons. During apheresis procedures, for example, blood is drawn from a patient or donor, a specific blood component is removed, and the remaining blood components are returned to the patient or donor. Apheresis procedures can be used to collect certain blood components (red blood cell, platelets, plasma, etc.) for donation, while also being useful in treating diseases. It is important during these procedures (and in any other procedure in which blood is drawn from a patient or donor) that there is proper blood flow. A low flow rate can result in a procedure taking a longer amount of time, while a high flow rate can result in vein collapse and/or may require operator interventions.

Apheresis and other blood draw procedures can range from approximately 30 minutes to 10 hours. During these procedures, patients/donors may be required to squeeze their arm to ensure proper blood flow. Due to the length of the procedure, this squeezing can result in arm fatigue and general discomfort.

It is known to provide tourniquets or inflatable cuffs to increase blood flow through a patient or donor's arm during a blood collection procedure. For example, PCT Patent Application Publication No. WO 2019/006349 A1 discloses a system for increasing blood flow that includes an inflatable cuff that also contains a heating element. US 4,883,462 refers to a blood extraction assist apparatus comprising a limb stimulator that is engaged with a lower portion of a patients limb for facilitating sustained venous blood flow. The stimulator may be an inflatable squeeze bulb adapted to engage a human hand and to be squeezed when inflated.

### Summary

The invention is set out in the appended set of claims. In one aspect, a blood flow assist device is provided. The device includes a flexible sleeve, at least one blood flow stimulator and an actuator assembly. The flexible sleeve has a first portion configured to receive at least a part of an arm of a donor or patient and a second portion configured to engage a portion of a hand of the patient or donor. The first and second portions of the flexible sleeve are configured to position the at least one blood flow stimulator at a target location of the arm. The at least one blood flow stimulator is incorporated into the flexible sleeve. The actuator assembly is coupled to at least one blood flow stimulator and configured to individually control and selectively actuate each blood flow stimulator to apply a stimulus to the arm to control blood flow through a vein in the arm or hand having a vein access device connected thereto.

In another aspect, there is provided a blood processing system. The blood processing system includes a blood flow assist device and a blood processing device. The blood flow assist device includes a flexible sleeve, a plurality of blood flow stimulators, and an actuator assembly. The flexible sleeve has a first portion configured to receive at least a part of an arm of a donor or patient and a second portion configured to engage a portion of a hand of the patient or donor. The plurality of blood flow stimulators are incorporated into the flexible sleeve with the first and second portions of the flexible sleeve being configured to position the at least one blood flow stimulator at a target location of the arm. The actuator assembly is configured to individually control and selectively actuate each blood flow stimulator to apply a stimulus to the arm to control blood flow through a vein in the arm or hand having a vein access device connected thereto. The blood processing device includes a controller, a blood draw pump configured to draw blood from the vein via the vein access device, and a pressure sensor. The controller is configured to receive a signal from the pressure sensor that is indicative of a capability of flow within the vein and to control operation of the blood draw pump and the actuator assembly based at least in part on said signal.

In another aspect, the blood flow assist device may be used in a method (not forming part of the invention as claimed) of controlling blood flow during a blood draw that includes associating a first portion of a flexible sleeve of a blood flow assist device to an arm of a patient or donor and a second portion of the flexible sleeve to a hand of the patient or donor, drawing blood from a vein of the arm, detecting a pressure at which the blood is being drawn from the arm or hand, and automatically applying a stimulus from the arm or hand using at least one blood flow stimulator of the blood flow assist device based at least in part on the pressure so as to modify a capability of flow within the vein, wherein the first and second portions of the flexible sleeve are configured to position the at least one blood flow stimulator at a target location of the arm.

### Brief Description of the Drawings

Fig. 1 is a schematic view of an exemplary blood flow assist device of the present disclosure;
Fig. 2 is a top plan view of the blood flow assist device of Fig. 1; and
Fig. 3 is a schematic view of a blood processing system including a blood processing device and the blood flow assist device of Figs. 1 and 2.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

While embodiments are described below in terms of use in an apheresis procedure, it will be appreciated that the devices, systems disclosed herein may be used in other types of blood collection or therapeutic procedures. Additionally, while the embodiments are described in terms of a sleeve that is worn around the arm of a patient or donor, the sleeve may alternatively (in not claimed embodiments) be worn around other limbs of the user. Furthermore, the terms "patient" and "donor" are used interchangeably in the following description.

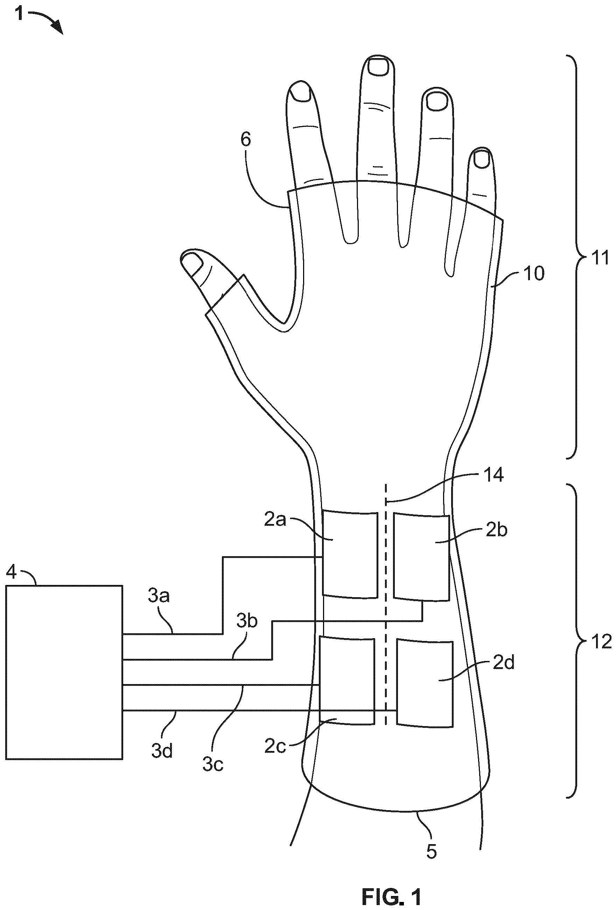

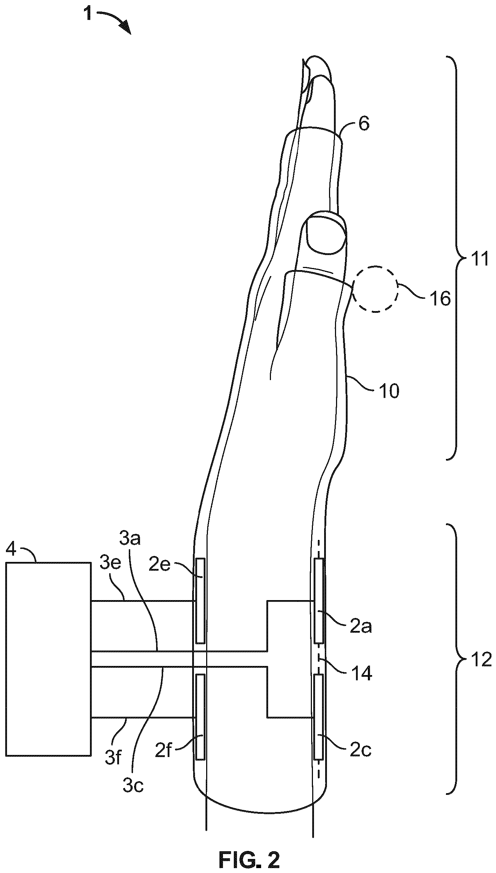

Figs. 1 and 2 show an exemplary embodiment of a blood flow assist device 1 of the current disclosure. The blood flow assist device 1 includes a sleeve 10, which is sized and dimensioned to be placed around an arm or other limb. In the illustrated embodiment, sleeve 10 extends over and around a portion of the patient's arm near the site of a blood draw (which may comprise a needle or vein access device positioned proximally of the sleeve 10, such as in the crease of the arm or positioned distally of the sleeve 10, such as in the top of the hand).

The sleeve 10 may be variously configured without departing from the scope of the present disclosure. For example, the sleeve 10 may be comprised of one or more layers of a flexible material to accommodate different limb sizes. The material can include fabric made from synthetic or natural fibers, preferably material that is soft and hypoallergenic. The fibers can be elastic, for example. The fibers can be woven or nonwoven. Fibers can be comprised of polyamides, polyesters, polyurethanes, vinyl, polyethylene, cellulose, silk, wool, rayon, cotton, nylon and any other known fibers in the art. If non-woven, the fibers can be stapled, melt-blown, spun-bond, or any other known method of bonding. The fiber and fabric can be elastic and considered stretch fabric. The sleeve layer or layers can be comprised of blends or combinations of any of the above materials. Different layers of the sleeve 10 can be comprised of the same material or material combinations or different materials or material combinations.

The sleeve 10 may be impregnated or coated with an antimicrobial or other beneficial agent. Antimicrobial agents for medical applications, including, but not limited to, zinc, silver, and chlorhexidine compounds can be utilized.

The sleeve 10 may be disposable or reusable and may be sterilized or disinfected before use. If reusable, it can be cleaned, sterilized, and/or disinfected before each use.

In one embodiment, the sleeve 10 is a single piece that can be pulled on and off without manipulating a fastening or closure element, in which case it may be especially advantageous for the sleeve 10 to formed of a flexible, elastic material. Alternatively, the sleeve 10 may include a fastening or closure element, in which case it may be less important for the sleeve 10 to be formed of an elastic material, though flexibility may still be advantageous. Exemplary fastening or closure elements include hook-and-loop fasteners, buckles, adhesives, zippers, clasps, drawstrings, and the like.

Regardless of the material composition and accessories of the sleeve 10, at least one blood flow stimulator is incorporated into the sleeve 10. While it is within the scope of the present disclosure for a single blood flow stimulator to be employed, it may be advantageous for a plurality of smaller blood flow stimulators to be employed to provide more control over a stimulus applied to the limb by the sleeve 10, as will be described in greater detail herein. In the illustrated embodiment and by way of example only, eight flood flow stimulators are incorporated into the sleeve 10, with four of the blood flow stimulators 2a-2d being visible in Fig. 1. The other four blood flow stimulators are incorporated into other portions of the sleeve 10, with Fig. 2 showing the possible positions of two such blood flow stimulators 2e and 2f.

Fig. 2 shows the blood flow stimulators 2 embedded within or positioned between two layers of the sleeve 10, but it should be understood that a blood flow stimulator 2 may be otherwise incorporated into the sleeve 10. For example, in other embodiments, a blood flow stimulator 2 may be located on an inner surface of the sleeve 10 (i.e., configured to be placed into direct contact with a limb received within the sleeve 10) or on an outer surface of the sleeve 10. The preferred location of a blood flow stimulator 2 may depend on the nature of stimulus applied to the limb by the blood flow stimulator 2, with certain blood flow stimulators being best positioned in contact with a limb and others not requiring direct contact.

The blood flow stimulators 2 can be arranged in a variety of ways without departing from the scope of the present disclosure. In an exemplary embodiment, at least two blood flow stimulators 2 are associated with the same surface or side of the sleeve 10. In the illustrated embodiment, the sleeve 10 includes first and second surfaces (Fig. 2), with four blood flow stimulators 2a-2d associated with one of the surfaces (Fig. 1) and four blood flow stimulators (including blood flow stimulators 2e and 2f) associated with the other surface. As shown in Fig. 2, one of the surfaces of the sleeve 10 (along with the associated blood flow stimulators 2) is configured to be positioned against an anterior surface of a limb, while the opposite surface of the sleeve 10 (along with the associated blood flow stimulators 2) is positioned against a posterior surface of the limb. While the illustrated embodiment includes blood flow stimulators 2 positioned against opposing surfaces of a limb, it should be understood that blood flow stimulators 2 may be positioned at other locations, such as stretching across surfaces of the sleeve 10 and extending along the full circumference of the sleeve 10.

Fig. 1 shows an exemplary configuration in which four blood flow stimulators 2a-2d associated with one of the first and second surfaces of the sleeve 10 are arranged in a square or rectangular or grid pattern. The blood flow stimulators 2 associated with the opposing surface of the sleeve 10 may be arranged in a different pattern or in a similar, mirror image pattern. In one embodiment, the sleeve 10 may be configured for placement on either the left or right limb (as will be described in greater detail herein), in which case it may be advantageous to provide the two opposing surfaces of the sleeve 10 with blood flow stimulators 2 arranged in mirror image patterns.

Regardless of the particular configuration and arrangement of the blood flow stimulators 2, they are designed to apply a stimulus to a limb so as to control blood flow through a vein having a vein access device (e.g., a needle) inserted therein. An individual blood flow stimulator 2 may be controlled so as to apply a substantially uniform stimulus to a limb during a blood draw procedure. Alternatively, a blood flow stimulator 2 may instead be controlled to apply a varying stimulus to a limb during a blood draw procedure. For example, in the case of a procedure having multiple phases, a blood flow stimulator 2 may apply one stimulus during one phase and a different stimulus during another phase. This may include providing no stimulus during one of the phases.

The nature of the stimuli applied by the blood flow stimulators 2 may vary without departing from the scope of the present disclosure. In one embodiment, the blood flow stimulators 2 are configured to apply pressure to a limb so as to control blood flow through a vein of the limb. This may be achieved by blood flow stimulators 2 configured as inflatable bladders, for example. Inflatable bladders of the current disclosure can be comprised of a single layer or layers of different materials. Inflatable bladders can be comprised of a variety of materials, including thermoplastic materials and elastomers, for example. Suitable materials can include polyethylene, polypropylene, polyurethanes, polyamides, polyesters, ethylene vinyl acetate, natural or synthetic polyisoprene, polybutadiene, polychloroprene, silicone, nitrile rubbers, nylon, olefin, and polyvinyl chloride. The bladder layer or layers can be comprised of blends or combinations of any of the above materials. Different layers of the bladder or bladders can be comprised of the same material or material combinations or different materials or material combinations.

The bladder or bladders may be impregnated or coated with an antimicrobial or other beneficial agent, particularly if they are configured to be placed into direct contact with a limb. Antimicrobial agents for medical applications, including, but not limited to, zinc, silver, and chlorhexidine compounds can be utilized.

Each bladder can have an associated pressure sensor for providing feedback as to the current pressure of each bladder and controlling the pressure applied to the limb by the bladder.

Pressure may also be applied by a mechanical assembly, such as rollers. The rollers may move in a single direction or multiple directions to stimulate muscles of the limb so as to control blood flow through a vein of the limb. The rollers or other parts of a mechanical assembly may also vibrate in order to stimulate the muscles of the limb.

As an alternative to pressure, stimuli may instead take the form of electrical stimulations. Electrical stimulation devices, such as electrodes, can be incorporated into the sleeve 10 and selectively actuated to stimulate muscles of the limb so as to control blood flow through a vein of the limb.

In addition to blood flow stimulators 2, the sleeve 10 may additionally include at least one heating element 14 or a plurality of heating elements. The heating element(s) 14 may be composed of various materials, including, but not limited to metallic or ceramic materials. The heating element(s) 14 can be ribbons or wire. The heating element 14 is shown in dotted line in Figs. 1 and 2 as a single heating element incorporated into the surface of the sleeve 10 positioned against the anterior surface of the limb, between the blood flow stimulators in a proximal section 12 of the sleeve 10. The heating element(s) can be arranged in various patterns across a section or entirety of the sleeve 10 and on either or both surfaces of the sleeve 10 positioned against the anterior and posterior surfaces of the limb.

An optional hand squeezing accessory 16 may also (or alternatively) be included as part of the sleeve 10 when the sleeve 10 is configured to be worn on an arm and hand. The hand squeezing accessory 16 may be a flexible material and may be formed into a ball or any other shape that is comfortably gripped or squeezed by hand. The optional hand squeezing accessory 16 is shown in Fig. 2 as a circular shape on a surface of the sleeve 10 in a distal section 11 of the sleeve 10. The hand squeezing accessory 16 can be attached by any conventional means and incorporated into or associated with the sleeve 10 at a suitable position (e.g., at a position overlaying the palm) so that it may be readily squeezed by the donor's hand during blood draw.

Actuation of the blood flow stimulators 2 may be controlled by an actuator assembly 4. For example, the blood flow stimulators 2 may be connected to the actuator assembly 4 by connectors 3. Each blood flow stimulator 2 can have its own connector 3, as shown in Figs. 1 and 2, or multiple blood flow stimulators 2 can be connected to the actuator assembly 4 by a single connector 3. Actuator assembly 4 may also control any included heating element(s) 14.

The nature and configurations of the actuator assembly 4 and connectors 3 may vary, depending on the nature and configurations of the blood flow stimulators 2. For example, if the blood flow stimulators 2 are configured as inflatable bladders, the actuator assembly 4 may be configured as an air control manifold having a source of fluid and a drive mechanism (e.g., a compressor or pump). Alternatively, a separate drive mechanism may be provided (e.g., incorporated into a blood draw system) and coupled to the actuator assembly 4. The connectors 3 may be configured as tubes formed of any of a variety of materials, including a polymeric material, such as a plastic or rubber polymer, polyvinyl chloride, polyethylene, or a combination of these. A valving arrangement of the actuator assembly 4 would direct inflating fluid (e.g., air or a liquid) into and out of the various bladders, allowing for individual control and actuation of the bladders.

If the blood flow stimulators are configured as a mechanical assembly, such as rollers, the actuator assembly 4 may be configured with various components such as a screw, cam, rod, spring and/or shaft and may be used in combination with a motor. A controller of the actuator assembly 4 may direct various components to move in combination to produce a rolling or vibrating motion.

On the other hand, if the blood flow stimulators 2 are configured as electrical stimulation devices or electrodes, the actuator assembly 4 may be configured as a generator or power source or source of electrical stimulation, with the connectors 3 configured as conductive wiring. A controller of the actuator assembly 4 may direct current to the various electrodes, allowing for individual control and actuation of the electrodes.

One device or a combination of devices can perform the functions of the actuator assembly 4. The actuator assembly 4 may be configured to receive power from an external power source or may feature an on-board battery. The actuator assembly 4 is preferably configured to operate the blood flow stimulators 2 within safe limits based on pressure feedback from pressure sensors or electrical sensors.

The actuator assembly 4 is fixedly or removably attached to the sleeve 10, and may be located on the sleeve 10 or separate from the sleeve 10. The actuator assembly 4 (under the control of an internal or separate controller) controls the actuation and timing of the blood flow stimulators 2. The blood flow stimulators 2 can be stimulated simultaneously or in succession. In one embodiment, a distally positioned blood flow stimulator 2 is actuated, followed by the sequential actuation of blood flow stimulators 2 each positioned proximally of the previously actuated blood flow stimulator 2. Such a sequence simulates the muscle response when a fist is being made to increase or enhance the blood flow in the vein. If blood flow stimulators 2 are positioned against both the anterior and posterior sides of the limb, the blood flow stimulators 2 on both sides of the limb may be actuated in such a sequence or the blood flow stimulators 2 on only one side of the limb (e.g., on the anterior side of the arm) may be actuated.

Properly stimulating a limb to control blood flow depends upon proper location of the various blood flow stimulators 2 with respect to the limb, to ensure that each blood flow stimulator 2 is applying a stimulus at a suitable location. To that end, the sleeve 10 may include a distal section 11 configured to be associated to a more distal portion or extremity 6 of the patient's limb and a proximal section 12 configured to be associated to a more proximal portion 5 of the patient's limb. In the illustrated embodiment, the distal section 11 of the sleeve 10 engages the hand, while the proximal section 12 surrounds a portion of the forearm or lower arm. In an alternative embodiment, the distal section 11 may be configured to engage a foot, while the proximal section 12 is configured to receive the lower leg or calf. The distal section 11 is configured to engage the extremity to prevent over-advancement or under-advancement of the sleeve 10 onto the limb. Figs. 1 and 2, for example, show the distal section 11 of the sleeve 10 configured as a fingerless glove, which fits onto the hand in a particular position that determines the positions of the blood flow stimulators 2 with respect to the arm. As different patients have different limb lengths, differently sized sleeves 10 may be provided, such as a smaller, shorter sleeve for a child and a larger, longer sleeve for an adult patient.

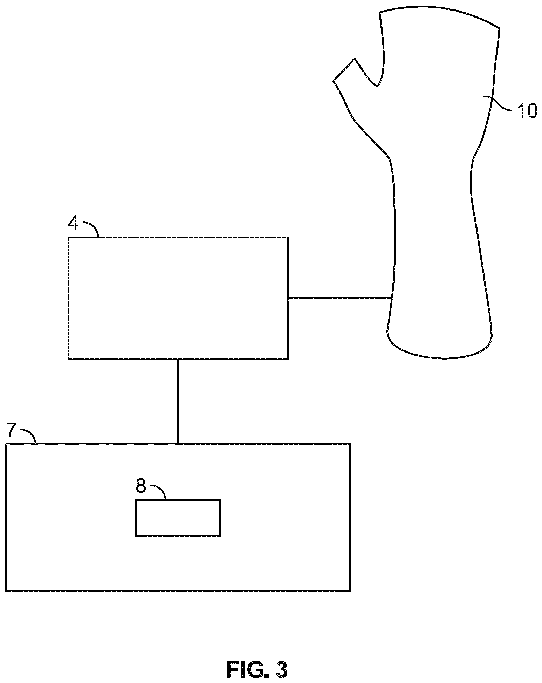

As described above, the actuator assembly 14 may be controlled by an internal or an external controller. Fig. 3 shows an arrangement in which the actuator assembly 4 is coupled to a blood draw device or system 7 (e.g., an apheresis device or system) having a controller 8 that controls operation of both the blood draw device 7 (or at least selected aspects thereof) and the actuator assembly 4. This connection can be wired or wireless. During blood draws, a needle or vein access device is placed into a vein of the patient's limb and is placed in fluid communication with the blood draw device 7. A blood draw pump of the blood draw device 7 is actuated by the controller 8 to draw blood into the blood draw device 7 from the vein, with pressure and/or flow rate being monitored and the controller 8 actuating the actuator assembly 4 and/or blood draw pump in response to pressure and/or flow rate levels.

When pressure is being monitored, the blood draw device 7 will include a pressure sensor (which may be variously configured without departing from the scope of the present disclosure) that monitors the input pressure of the blood draw pump, which is indicative of vein pressure and the capability of flow within the vein. When the input pressure is greater than a maximum or target pressure, the controller 8 will actuate the actuator assembly 4 to increase the stimulus applied in an attempt to increase the capability of flow within the vein and decrease the input pressure. The controller 8 may also generate an alert in the event of an elevated input pressure. The controller 8 may further control the operation of the blood draw pump based on the input pressure and/or an output pressure of the blood draw pump. For example, if the input pressure is above the target pressure, the controller 8 may command the blood draw pump to operate a lower rate in order to reduce the risk of vein collapse.

The hand squeezing accessory 16 can also be connected (wired or wirelessly) to the blood draw device or system 7. The blood draw device or system 7 can prompt the donor to squeeze the hand squeezing accessory when needed.

As for flow rate monitoring, either a direct blood flow sensor or an indirect approach may be employed. In one exemplary embodiment of an indirect approach, flow rate may be calculated based on the number of pump head rotations and the amount of fluid delivered to a particular receptacle or location (e.g., using a weight scale associated with a fluid container. If the draw rate is below a target rate or a target range, the controller 8 may control the actuator assembly 4 to stimulate the limb so as to increase the capability of flow within the vein. On the other hand, if the draw rate is above a target rate or a target range, the controller 8 may control the actuator assembly 4 to reduce or remove stimuli being applied to the limb. The blood draw device 7 may also monitor vein pressure and reduce or remove stimuli being applied to the limb to prevent vein collapse, as described above. In addition to a blood draw phase, a procedure executed by the blood draw device may have a variety of phases. In this case, the controller 8 and actuator assembly 4 can adjust the stimuli based on the phase of the procedure.

## Claims

1. A blood flow assist device (1), comprising:
a flexible sleeve (10) with a first portion (11) configured to receive at least a part of an arm of a patient or donor and a second portion (12) configured to engage a portion of a hand of the patient or donor, the flexible sleeve including opposing first and second surfaces configured to be positioned against opposing anterior and posterior surfaces of the arm when said at least a portion of the arm is received within the flexible sleeve;
a first plurality of blood flow stimulators (2) incorporated into the first surface of the flexible sleeve and a second plurality of blood flow stimulators (2) incorporated into the second surface of the flexible sleeve; and
an actuator assembly (4) coupled to the first and second plurality of blood flow stimulators, wherein
the actuator assembly is configured to individually control and selectively actuate each blood flow stimulator to apply a stimulus to the arm to control blood flow through a vein in the arm or hand having a vein access device connected thereto,
the first and second portions of the flexible sleeve are configured to position the first and second plurality of blood flow stimulators at a target location of the arm,
the flexible sleeve is configured to receive the left arm of a patient or donor to position the first plurality of blood flow stimulators against the anterior surface of the left arm and the second plurality of blood flow stimulators against the posterior surface of the left arm, and
the flexible sleeve is configured to receive the right arm of a patient or donor to position the second plurality of blood flow stimulators against the anterior surface of the right arm and the first plurality of blood flow stimulators against the posterior surface of the right arm.

2. The blood flow assist device of claim 1, wherein the actuator assembly is configured to control and actuate said first and second plurality of blood flow stimulators to apply stimuli to the arm of a patient or donor so as to simulate the condition of muscles in the arm when making a fist with the hand.

3. The blood flow assist device of any one of the preceding claims, wherein the second plurality of blood flow stimulators are incorporated into the flexible sleeve in a pattern that is a mirror image of a pattern in which the first plurality of blood flow stimulators are incorporated into the flexible sleeve.

4. The blood flow assist device of any one of the preceding claims, wherein the actuator assembly is configured to actuate each blood flow stimulator differently when said blood flow stimulator is positioned against the anterior surface of the arm and when said blood flow stimulator is positioned against the posterior surface of the arm.

5. The blood flow assist device of any one of the preceding claims, wherein the actuator assembly is configured to actuate the first and second plurality of blood flow stimulators to apply a substantially uniform stimulus to the arm during a blood draw procedure.

6. The blood flow assist device of any one of claims 1-4, wherein the actuator assembly is configured to actuate the first and second plurality of blood flow stimulators to apply a varying stimulus to the arm during a blood draw procedure.

7. The blood flow assist device of any one of the preceding claims, wherein the actuator assembly is configured to actuate one of the blood flow stimulators to apply a different stimulus to the arm than another one of the blood flow stimulators during a blood draw procedure.

8. The blood flow assist device of any one of the preceding claims, wherein at least one of the blood flow stimulators comprises an inflatable bladder or electrode.

9. The blood flow assist device of any one of the preceding claims, further comprising at least one heating element (14).

10. The blood flow assist device of any one of the preceding claims, further comprising a hand squeezing accessory (16).

11. A blood processing system including a blood flow assist device according to any one of the preceding claims and further including a blood processing device (7) including
a blood draw pump configured to draw blood from the vein via said vein access device,
a pressure sensor, and
a controller (8) configured to receive a signal from the pressure sensor that is indicative of a capability of flow within the vein and to control operation of the blood draw pump and the actuator assembly based at least in part on said signal.

12. The blood processing system of claim 11, wherein the controller is configured to
compare the pressure at which blood is being drawn into the blood processing device from the arm or hand to a target pressure, and
control the actuator assembly to increase the stimulus applied to the arm by at least one of the blood flow stimulators when the pressure at which blood is being drawn into the blood processing device from the arm or hand is greater than the target pressure, preferably generating an alert when the pressure at which blood is being drawn into the blood processing device from the arm or hand is greater than the target pressure.

13. The blood processing system of any one of claims 11-12, wherein the controller is configured to
control the blood processing device to execute a procedure having a plurality of phases,
control the actuator assembly to apply stimulus to the arm during one phase of the procedure, and
control the actuator assembly to apply stimulus to the arm during another phase of the procedure that are different from the stimulus applied to the arm during said one phase of the procedure.

14. The blood processing system of claim 13, wherein the controller is configured to control the actuator assembly to apply no stimulus to the arm during one of the phases of the procedure.

## Patentansprüche

1. Vorrichtung (1) zur Unterstützung des Blutflusses, umfassend:
eine flexible Hülle (10) mit einem ersten Abschnitt (11) zum Aufnehmen zumindest von einem Teil eines Arms eines Patienten oder Spenders und mit einem zweiten Abschnitt (12) zum Ineingriffbringen mit einem Teil einer Hand des Patienten oder Spenders, wobei die flexible Hülle eine erste und eine zweite Fläche aufweist, die einander gegenüberliegen und dazu ausgelegt sind, an eine vordere und eine dieser gegenüberliegende hintere Fläche des Arms positioniert zu werden, wenn der zumindest eine Teil des Arms in der flexiblen Hülle aufgenommen ist;
eine erste Vielzahl von Blutflussstimulatoren (2), die in der ersten Fläche der flexiblen Hülle eingebettet sind, und eine zweite Vielzahl von Blutflussstimulatoren (2), die in der zweiten Fläche der flexiblen Hülle eingebettet sind; und
eine Betätigungsanordnung (4), die mit der ersten und der zweiten Vielzahl von Blutflussstimulatoren gekoppelt ist, wobei
die Betätigungsanordnung dazu ausgelegt ist, jeden Blutflussstimulator einzeln anzusteuern und selektiv zu betätigen, um einen Reiz auf den Arm auszuüben, um den Blutfluss durch eine Vene im Arm oder in der Hand zu beeinflussen, an die eine Venenzugangsvorrichtung angeschlossen ist,
der erste und der zweite Abschnitt der flexiblen Hülle dazu ausgelegt sind, die erste und die zweite Vielzahl von Blutflussstimulatoren an einer Zielstelle am Arm zu positionieren,
die flexible Hülle dazu ausgelegt ist, den linken Arm eines Patienten oder Spenders aufzunehmen, um die erste Vielzahl von Blutflussstimulatoren an der vorderen Fläche des linken Arms und die zweite Vielzahl von Blutflussstimulatoren an der hinteren Fläche des linken Arms zu positionieren,
die flexible Hülle dazu ausgelegt ist, den rechten Arm eines Patienten oder Spenders aufzunehmen, um die zweite Vielzahl von Blutflussstimulatoren an der vorderen Fläche des rechten Arms und die erste Vielzahl von Blutflussstimulatoren an der hinteren Fläche des rechten Arms zu positionieren.

2. Vorrichtung zur Unterstützung des Blutflusses nach Anspruch 1, wobei die Betätigungsanordnung dazu ausgelegt ist, die erste und die zweite Vielzahl von Blutflussstimulatoren anzusteuern und zu betätigen, um Reize auf den Arm eines Patienten oder Spenders auszuüben, um den Zustand der Muskeln im Arm zu simulieren, wenn mit der Hand eine Faust gemacht wird.

3. Vorrichtung zur Unterstützung des Blutflusses nach einem der vorhergehenden Ansprüche, wobei die zweite Vielzahl von Blutflussstimulatoren in der flexiblen Hülle in einem Muster eingebettet sind, das ein Spiegelbild eines Musters ist, in dem die erste Vielzahl von Blutflussstimulatoren in der flexiblen Hülle eingebettet sind.

4. Vorrichtung zur Unterstützung des Blutflusses nach einem der vorhergehenden Ansprüche, wobei die Betätigungsanordnung dazu ausgelegt ist, jeweils den Blutflussstimulator unterschiedlich zu betätigen, je nachdem, ob der Blutflussstimulator an der vorderen Fläche des Arms positioniert ist oder ob der Blutflussstimulator an der hinteren Fläche des Arms positioniert ist.

5. Vorrichtung zur Unterstützung des Blutflusses nach einem der vorhergehenden Ansprüche, wobei die Betätigungsanordnung dazu ausgelegt ist, die erste und die zweite Vielzahl von Blutflussstimulatoren zu betätigen, um während eines Blutentnahmevorgangs einen im Wesentlichen gleichmäßigen Reiz auf den Arm auszuüben.

6. Vorrichtung zur Unterstützung des Blutflusses nach einem der Ansprüche 1 bis 4, wobei die Betätigungsanordnung dazu ausgelegt ist, die erste und die zweite Vielzahl von Blutflussstimulatoren zu betätigen, um während eines Blutentnahmevorgangs einen variierenden Reiz auf den Arm auszuüben.

7. Vorrichtung zur Unterstützung des Blutflusses nach einem der vorhergehenden Ansprüche, wobei die Betätigungsanordnung dazu ausgelegt ist, einen der Blutflussstimulatoren zu betätigen, um während eines Blutentnahmevorgangs einen anderen Reiz auf den Arm auszuüben als ein anderer der Blutflussstimulatoren.

8. Vorrichtung zur Unterstützung des Blutflusses nach einem der vorhergehenden Ansprüche, wobei zumindest einer der Blutflussstimulatoren einen aufblasbaren Ballon oder eine Elektrode umfasst.

9. Vorrichtung zur Unterstützung des Blutflusses nach einem der vorhergehenden Ansprüche, ferner umfassend zumindest ein Heizelement (14).

10. Vorrichtung zur Unterstützung des Blutflusses nach einem der vorhergehenden Ansprüche, ferner umfassend ein Zubehörteil (16) zum Zusammendrücken mit der Hand.

11. Blutbehandlungssystem mit einer Vorrichtung zur Unterstützung des Blutflusses nach einem der vorhergehenden Ansprüche und ferner mit einer Blutbehandlungsvorrichtung (7), umfassend
eine Blutentnahmepumpe zum Entnehmen von Blut aus der Vene über die Venenzugangsvorrichtung,
einen Drucksensor, und
eine Steuerung (8), die dazu ausgelegt ist, ein Signal von dem Drucksensor zu empfangen, das eine Strömungsfähigkeit in der Vene anzeigt, und den Betrieb der Blutentnahmepumpe und der Betätigungsanordnung zumindest teilweise auf der Grundlage des Signals zu steuern.

12. Blutbehandlungssystem nach Anspruch 11, wobei die Steuerung ausgelegt ist zum
Vergleichen des Drucks, mit dem Blut aus dem Arm oder der Hand in die Blutbehandlungsvorrichtung geleitet wird, mit einem Solldruck, und
Ansteuern der Betätigungsanordnung, um den von zumindest einem der Blutflussstimulatoren auf den Arm ausgeübten Reiz zu erhöhen, wenn der Druck, mit dem Blut aus dem Arm oder der Hand in die Blutbehandlungsvorrichtung geleitet wird, höher als der Solldruck ist, und vorzugsweise Erzeugen eines Alarms, wenn der Druck, mit dem Blut aus dem Arm oder der Hand in die Blutbehandlungsvorrichtung geleitet wird, höher als der Solldruck ist.

13. Blutbehandlungssystem nach einem der Ansprüche 11 bis 12, wobei die Steuerung ausgelegt ist zum
Ansteuern der Blutbehandlungsvorrichtung, um einen Prozess mit mehreren Phasen auszuführen,
Ansteuern der Betätigungsanordnung, um während einer Prozessphase einen Reiz auf den Arm auszuüben, und
Ansteuern der Betätigungsanordnung, um während einer anderen Prozessphase einen Reiz auf den Arm auszuüben, der sich von dem Reiz unterscheidet, der während der einen Prozessphase auf den Arm ausgeübt wird.

14. Blutbehandlungssystem nach Anspruch 13, wobei die Steuerung dazu ausgelegt ist, die Betätigungsanordnung anzusteuern, um während einer der Prozessphasen keinen Reiz auf den Arm auszuüben.

## Revendications

1. Dispositif d'aide au flux sanguin (1), comprenant :
un manchon flexible (10) avec une première partie (11) configurée pour recevoir au moins une partie d'un bras d'un patient ou d'un donneur et une seconde partie (12) configurée pour venir en prise avec une partie d'une main du patient ou du donneur, le manchon flexible comportant des première et seconde surfaces opposées configurées pour être positionnées contre des surfaces antérieures et postérieures opposées du bras lorsque ladite au moins une partie du bras est reçue à l'intérieur du manchon flexible ;
une première pluralité de stimulateurs de flux sanguin (2) incorporée dans la première surface du manchon flexible et une deuxième pluralité de simulateurs de flux sanguin (2) incorporée dans la seconde surface du manchon flexible ; et
un ensemble actionneur (4) couplé à la première et deuxième pluralité de stimulateurs de flux sanguin, dans lequel
l'ensemble actionneur est configuré pour commander individuellement et actionner sélectivement chaque stimulateur de flux sanguin pour appliquer un stimulus au bras pour commander du flux sanguin à travers une veine dans le bras ou la main présentant un dispositif d'accès à la veine relié à celui-ci ou celle-ci, et
les première et seconde parties du manchon flexible sont configurées pour positionner les première et deuxième pluralités de stimulateurs de flux sanguin sur un emplacement cible du bras,
le manchon flexible est configuré pour recevoir le bras gauche d'un patient ou d'un donneur pour positionner la première pluralité de stimulateurs de flux sanguin contre la surface antérieure du bras gauche et la deuxième pluralité de stimulateurs de flux sanguin contre la surface postérieure du bras gauche, et
le manchon flexible est configuré pour recevoir le bras droit d'un patient ou d'un donneur pour positionner la deuxième pluralité de stimulateurs de flux sanguin contre la surface antérieure du bras droit et la première pluralité de stimulateurs de flux sanguin contre la surface postérieure du bras droit.

2. Dispositif d'aide au flux sanguin selon la revendication 1, dans lequel l'ensemble actionneur est configuré pour commander et actionner lesdites première et deuxième pluralités de stimulateurs de flux sanguin pour appliquer des stimuli au bras d'un patient ou d'un donneur de manière à simuler l'état des muscles dans le bras lors de la formation d'un poing avec la main.

3. Dispositif d'aide au flux sanguin selon l'une quelconque des revendications précédentes, dans lequel la deuxième pluralité de stimulateurs de flux sanguin sont incorporés dans le manchon flexible selon un motif qui est une image miroir d'un motif dans lequel la première pluralité de stimulateurs de flux sanguin sont incorporés dans le manchon flexible.

4. Dispositif d'aide au flux sanguin selon l'une quelconque des revendications précédentes, dans lequel l'ensemble actionneur est configuré pour actionner chaque stimulateur de flux sanguin différemment lorsque ledit stimulateur de flux sanguin est positionné contre la surface antérieure du bras et lorsque ledit stimulateur de flux sanguin est positionné contre la surface postérieure du bras.

5. Dispositif d'aide au flux sanguin selon l'une quelconque des revendications précédentes, dans lequel l'ensemble actionneur est configuré pour actionner les première et deuxième pluralités de stimulateurs de flux sanguin pour appliquer un stimulus sensiblement uniforme au bras pendant une procédure de prélèvement sanguin.

6. Dispositif d'aide au flux sanguin selon l'une quelconque des revendications 1 - 4, dans lequel l'ensemble actionneur est configuré pour actionner les première et deuxième pluralités de stimulateurs de flux sanguin pour appliquer un stimulus variable au bras pendant une procédure de prélèvement sanguin.

7. Dispositif d'aide au flux sanguin selon l'une quelconque des revendications précédentes, dans lequel l'ensemble actionneur est configuré pour actionner un des stimulateurs de flux sanguin pour appliquer un stimulus différent au bras qu'un autre des stimulateurs de flux sanguin pendant une procédure de prélèvement sanguin.

8. Dispositif d'aide au flux sanguin selon l'une quelconque des revendications précédentes, dans lequel au moins un des stimulateurs de flux sanguin comprend une vessie ou électrode gonflable.

9. Dispositif d'aide au flux sanguin selon l'une quelconque des revendications précédentes, comprenant en outre au moins un élément chauffant (14).

10. Dispositif d'aide au flux sanguin selon l'une quelconque des revendications précédentes, comprenant en outre un accessoire de compression de main (16).

11. Système de traitement du sang comportant un dispositif d'aide au flux sanguin selon l'une quelconque des revendications précédentes et comportant en outre un dispositif de traitement du sang (7) comportant
une pompe de prélèvement sanguin configurée pour prélever du sang dans la veine par l'intermédiaire dudit dispositif d'accès à la veine,
un capteur de pression, et
un contrôleur (8) configuré pour recevoir un signal du capteur de pression qui indique une capacité d'écoulement à l'intérieur de la veine et pour commander le fonctionnement de la pompe de prélèvement sanguin et de l'ensemble actionneur sur la base au moins en partie dudit signal.

12. Système de traitement du sang selon la revendication 11, dans lequel le contrôleur est configuré pour
comparer la pression à laquelle du sang est prélevé dans le dispositif de traitement du sang depuis le bras ou la main à une pression cible, et
commander l'ensemble actionneur pour augmenter le stimulus appliqué au bras par au moins un des stimulateurs de flux sanguin lorsque la pression à laquelle du sang est prélevé dans le dispositif de traitement du sang depuis le bras ou la main est supérieure à la pression cible, générant de préférence une alerte lorsque la pression à laquelle du sang est prélevé dans le dispositif de traitement du sang depuis le bras ou la main est supérieure à la pression cible.

13. Système de traitement du sang selon l'une quelconque des revendications 11-12, dans lequel le contrôleur est configuré pour
commander le dispositif de traitement du sang pour exécuter une procédure présentant une pluralité de phases,
commander l'ensemble actionneur pour appliquer un stimulus au bras pendant une phase de la procédure, et
commander l'ensemble actionneur pour appliquer un stimulus au bras pendant une autre phase de la procédure qui est différent du stimulus appliqué au bras pendant ladite une phase de la procédure.

14. Système de traitement du sang selon la revendication 13, dans lequel le contrôleur est configuré pour commander l'ensemble actionneur pour n'appliquer aucun stimulus pendant une des phases de la procédure.
